(19) Europäisches Patentamt / European Patent Office / Office européen des brevets

(11) **EP 2 765 197 A1**

(12) **EUROPEAN PATENT APPLICATION**

(43) Date of publication:
**13.08.2014 Bulletin 2014/33**

(51) Int Cl.:
***C12N 15/63*** [(2006.01)]

(21) Application number: **13000700.8**

(22) Date of filing: **11.02.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **ETH Zurich**
**8092 Zurich (CH)**

(72) Inventors:
• **Auslaender, David**
  **CH - 4058 Basel (CH)**
• **Auslaender, Simon**
  **CH - 4058 Basel (CH)**
• **Fussenegger, Martin**
  **CH - 5506 Mägenwil (CH)**

(54) **Designer pH sensor as universal transgene controller**

(57) The invention relates to vectors and mammalian cells in a system useful for switching on or switching off gene expression in response to pH and gaseous carbon dioxide, taking advantage of signal transduction of the human proton-activated cell-surface receptor TDAG8, OGR1 and/or GPR4 and related receptors to chimeric promoters containing cAMP-response elements. The systems according to the invention can be used to precisely monitor culture pH within the physiologic range, or also to adjust expression by gaseous $CO_2$ which shifts the $CO_2$-bicarbonate balance towards hydrogen ions. Production of biopharmaceuticals of engineered cells cultivated in a bioreactor setup can be induced by gaseous CO2. Also, implanting engineered cells provide novel treatment strategies for acidosis-related disorders and type 1 diabetes.

**Fig. 1**

**Description**

**Field of the invention**

The invention relates to vectors and mammalian cells in a system useful for switching on or switching off gene expression in response to pH and gaseous carbon dioxide, a bioprocessing method for the $CO_2$-inducible production of biopharmaceuticals and a therapeutic method of treating acidosis-related disorders and type 1 diabetes.

**Background of the invention**

Synthetic biology is the science of reassembling catalogued and standardized biological items in a systematic and rational manner to create and engineer functional biological designer devices, systems and organisms with novel and useful functions (Weber W. and Fussenegger M., 2012, Nat Rev Genet 13, 21-35). During the past decade mammalian synthetic biology has progressed from simple control switches providing trigger-inducible transgene expression to complex transcription/translation networks enabling oscillating expression dynamics, intercellular communication and fundamental arithmetic operations. To date, biomedical applications for the treatment of bacterial infections, dengue hemorrhagic fever and malaria and cancer have passed proof-of-concept level, and the first synthetic biology-inspired treatment strategies have been successfully tested in animal models of prominent human disorders including conception challenges, obesity, T-cell therapy, type 2 diabetes, gouty arthritis, and the metabolic syndrome.

Type 1 diabetes mellitus is a chronic metabolic disease that is characterized by excessive blood glucose levels. While the precise risk and trigger factors remain elusive there is consensus that type 1 diabetes is a polygenic disorder, whose pathophysiology includes complete autoimmune destruction of the insulin-producing beta cells in the islets of Langerhans of the pancreas. Since there is no cure available yet, current treatment strategies include lifelong insulin replacement therapy by subcutaneous injection or insulin pumps along with precise dietary management and careful monitoring of blood glucose levels. This requires training, appropriate care and discipline in testing and dosing which is particularly challenging for children, disabled and elderly patients and during intercurrent infection-related disturbances of insulin levels. Although pancreas and islet cell transplantation have also been successfully established, these therapies are considered dangerous, require a lot of donor material and essentially replace lifelong insulin therapy by lifelong treatment with immunosuppressive agents. Poorly managed glucose homeostasis may result in a variety of complications including cardiovascular disease, diabetic neuropathy, diabetic retinopathy as well as ketoacidosis (Hovorka R., 2011, Nat Rev Endocrinol 7, 385-395).

When the glucose in glycogen stores cannot be mobilized and metabolized due to insulin deficiency the body activates its fat stores by actuating lipolysis and providing fatty acids as an alternative energy source. Muscle and liver cells break down fatty acids into acetyl-CoA to generate metabolic energy via the tricarboxylic acid (TCA) cycle. Since long-chain fatty acids are bound to albumin in the blood they cannot cross the blood-brain barrier. Therefore, in a process known as ketosis, the liver manages the energy supply chain for the brain by producing acetyl-CoA-derived ketone bodies (acetoacetate and 3-hydroxybutyrate) which cross the blood-brain barrier and are remanufactured to acetyl-CoA for TCA cycle-based energy production in the brain. Unless counteracted by insulin, which switches from fat- to glucose-based energy supplies, excess acidic ketone bodies accumulate in the blood, overwhelm the acid-base homeostasis maintained by the $CO_2$-bicarbonate buffering system and produce any medical condition ranging from mild acidosis (pH<7.35) to acute ketoacidosis leading to cerebral edema, coma and death (pH<7.1) (Kitabchi A.E. et al., 2001, Diabetes Care 24, 131-153).

Protons are an intrinsic part of $H_2O$, and since life arose and still operates in aqueous solutions it is no surprise that pH is a key parameter that impacts protein folding, stability, protein-protein interactions and activity and therefore needs to be reliably kept within a narrow biochemically permissive range (e.g., pH 7.35-7.45 in humans) (Casey J.R. et al., 2010, Nat Rev Mol Cell Biol 11, 50-61). The $CO_2$-bicarbonate buffering system manages acid-base homeostasis and maintains almost constant pH in all living systems including humans. In a completely reversible reaction catalyzed by carbonic anhydrases gaseous $CO_2$ reacts with $H_2O$ to form carbonic acid, which rapidly dissociates into bicarbonate and hydrogen ions. Increases in $CO_2$ resulting from oxidative metabolism are balanced by elimination of gaseous $CO_2$ through alveolar ventilation. Because of the strict correlation between $H^+$ and $CO_2$ in aqueous solutions, $CO_2$ can shift this balance towards high proton concentration and so critically influences culture, tissue and body pH. Therefore, gaseous $CO_2$ is also used to control pH in cell culture systems and biopharmaceutical manufacturing in a traceless and non-invasive manner. Biopharmaceuticals have a prominent position in today's therapeutic intervention portfolio and provide unique treatment opportunities for millions of patients worldwide suffering from severe diseases such as cancer. For economic and FDA-licensed manufacturing most blockbuster biopharmaceuticals are produced in protein-free chemically-defined mammalian suspension cultures grown in high-volume stirred-tank bioreactors (Aggarwal S., 2011, Nature Biotechnology 29, 1083-1089). Mammalian cell-based production of biopharmaceuticals reaches close to 1% of the worlds GDP worth 100 billion USD, and includes blockbuster drugs such as Rituximab, a chimeric monoclonal anti-CD20 immunoglobulin (IgG) that is licensed for the treatment of diseases characterized by excessive, overactive or dysfunctional B cells such as lymphomas, leukemias, transplant rejection and certain types of autoimmune disorders.

Specialized human cells express a variety of extracellular and intracellular acid-sensing proteins that monitor pH and adapt physiologic activities to pH changes. For example, members of the acid-sensing ion channels (ASICs) expressed in primary sensory neurons modulate pain, transient receptor potential (TRP) ion channels function as sour taste receptors, and a group of G-protein-coupled receptors (GPCRs) such as ovarian cancer G protein-coupled receptor 1 (OGR1), GPR4 and T cell death-associated gene 8 (TDAG8) are expressed in various cell types including immune, nerve and bone cells, but their functions are not yet clearly established (Ishii S. et al., 2005, J Biol Chem 280, 9083-9087). These proton-sensing GPCRs monitor pH via histidine residues in their extracellular domain. Since TDAG8 transcripts have been identified predominantly in nerve and immune cells the receptor has been suggested to modulate pain reactions and immune responses but its precise physiological function remains elusive.

**Summary of the invention**

The invention relates to a mammalian cell comprising

(a) a vector expressing a pH sensing receptor comprising a promoter and a polynucleotide coding for a G protein-coupled receptor;
(b) a vector comprising a synthetic promoter specifically activated by said pH sensing receptor and response elements fused to a polynucleotide coding for an endogenous or exogenous protein.

In particular the invention relates to such a mammalian cell wherein the G protein-coupled receptor is TDAG8.

The invention likewise relates to a vector comprising a synthetic promoter specifically activated by a pH sensing G protein-coupled receptor and response elements fused to a polynucleotide coding for an endogenous or exogenous protein.

Furthermore the invention relates to a bioprocessing method for the $CO_2$-inducible production of biopharmaceuticals wherein a mammalian cell of the invention expressing the biopharmaceutical is cultured in a bioreactor with pH control by introducing $CO_2$.

The invention also relates to the mentioned mammalian cell in a nano- or microcontainer, a mammal excluding man comprising such a mammalian cell optionally in a nano- or microcontainer, and to a method of treating acidosis-related disorders and type 1 diabetes administering such vectors and/or cells.

**Brief description of the Figures**

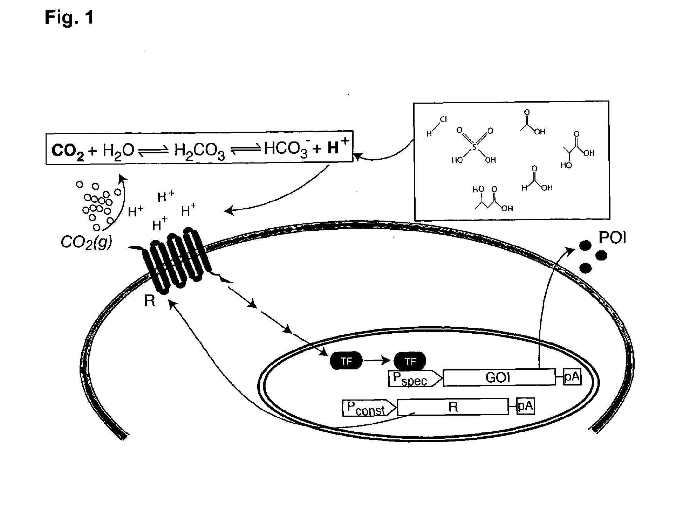

_Figure_ 1: _Design_ of _the synthetic pH-Sensor device_
A constitutively expressed pH-sensitive G protein-coupled receptor (R), e.g. TDAG8, OGR1 or GPR4, senses extracellular proton levels and triggers a pH-adjusted activation of an intracellular signalling cascade (→→→). The activated signalling cascade can be rewired to actuate specific transcription factors (TF) which bind and induce synthetic promoters (Pspec), e.g. CRE, SRE, NFAT or TRE. Subsequently, transcription of transgenes (gene of interest, GOI) in response to pH changes results either from shifts in the $CO_2$-bicarbonate buffering system induced by gaseous carbon dioxide ($CO_2$) or by addition of (in)organic acids and produces the protein of interest (POI).

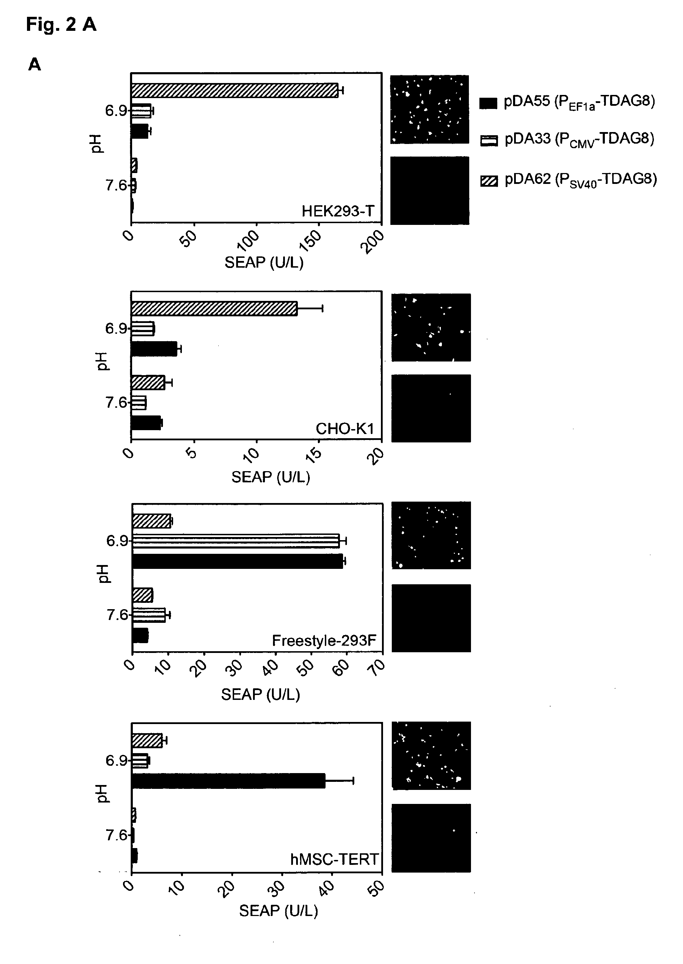

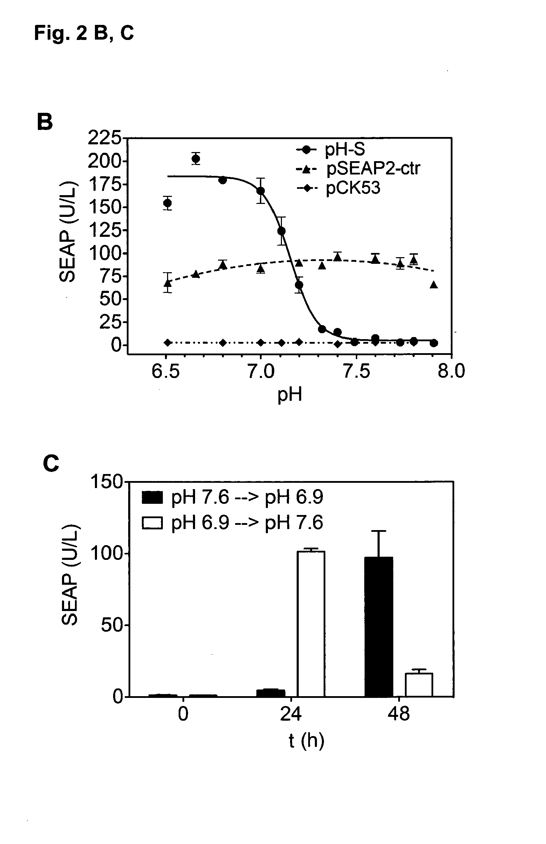

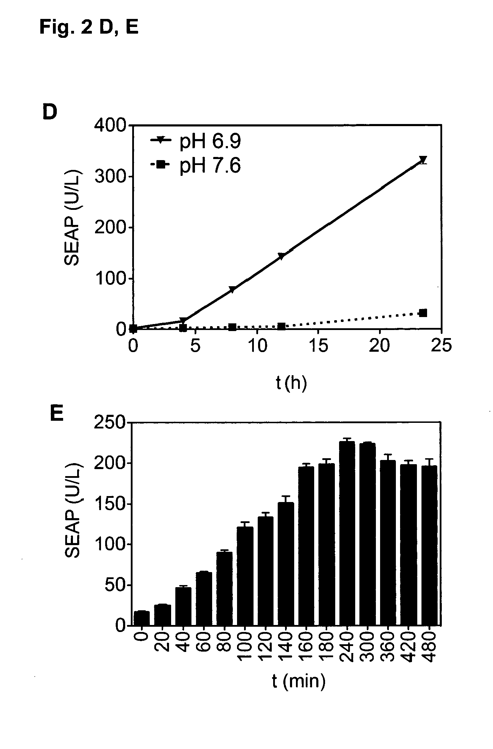

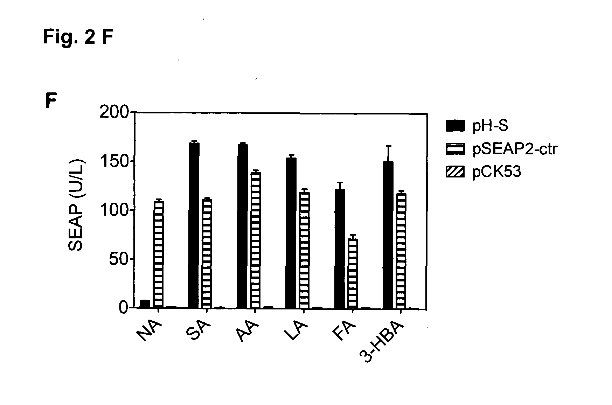

_Figure_ 2: _Characterization_ of _the pH sensor_

(A) pH sensor performance in different mammalian cell lines. HEK-293T, CHO-K1, _Freestyle™_-293F and hMSC-TERT were cotransfected with a constitutive TDAG8 expression vector (pDA55, $P_{hEF1\alpha}$-TDAG8-pA; pDA33, $P_{hCMV}$-TDAG8-pA; pDA62, $P_{SV40}$-TDAG8-pA) as well as a $P_{CRE}$-driven SEAP expression plasmid (pCK53, $P_{CRE}$-SEAP-pA) and exposed to high (7.6) or low (6.9) physiologic pH for 24 h before SEAP expression was quantified in the culture supernatant. Likewise, the best-in-class TDAG8 expression vector of each cell line (pDA62, HEK-293T/CHO-K1; pDA55, _Freestyle™_-293F/hMSC-TERT) was cotransfected with a $P_{CRE}$-driven EYFP expression plasmid (pCK91, $P_{CRE}$-EYFP-pA) and reporter gene expression was visualized by fluorescence microscopy after a 24 h cultivation period at high and low pH.
(B) Sensitivity and adjustability of the pH sensor (pH-S). HEK-293T were (co-)transfected with pH sensor encoding vectors (pDA62/pCK53), a constitutive SEAP expression plasmid (pSEAP2-ctr) or the $P_{CRE}$-driven SEAP reporter construct (pCK53) and cultivated for 24 h in medium adjusted to different pH values before SEAP was profiled in the culture supernatant. ($EC_{50}$ = pH 7.15 $\pm$ 0.03).

(C) Reversibility of the pH sensor. The reversibility of pH sensor driven SEAP expression was assessed by cultivating pDA62-/pCK53-cotransfected HEK-293T for 48 h in medium adjusted to high (7.6) or low (6.9) pH while resetting the cell density to $1x10^5$ cells/ml and alternating the culture pH after 24 h.

(D) pH sensor induction kinetics. HEK-293T cells cotransfected with pDA62 and pCK53 were grown in medium adjusted to high (7.6) or low (6.9) pH and SEAP expression was profiled for 24 h.

(E) Programming pH sensor induction kinetics. SEAP expression profiles of pDA62/- pCK53-cotransfected HEK-293T cultivated for different periods of time in low pH (6.9) culture medium.

(F) Sensitivity of the pH sensor to different acid loads. HEK-293T (co-)transfected with pH sensor components (pDA62/pCK53), pSEAP2-control, or the $P_{CRE}$-driven SEAP reporter vector (pCK53) were cultivated for 24 h in medium acidified to pH6.9 using different (in)organic acids before SEAP expression was assessed in the cell culture supernatant. NA = no acid, SA = sulphuric acid, AA = acetic acid, LA = lactic acid, FA = formic acid, 3-zBOH = 3-hydroxybutyric acid.

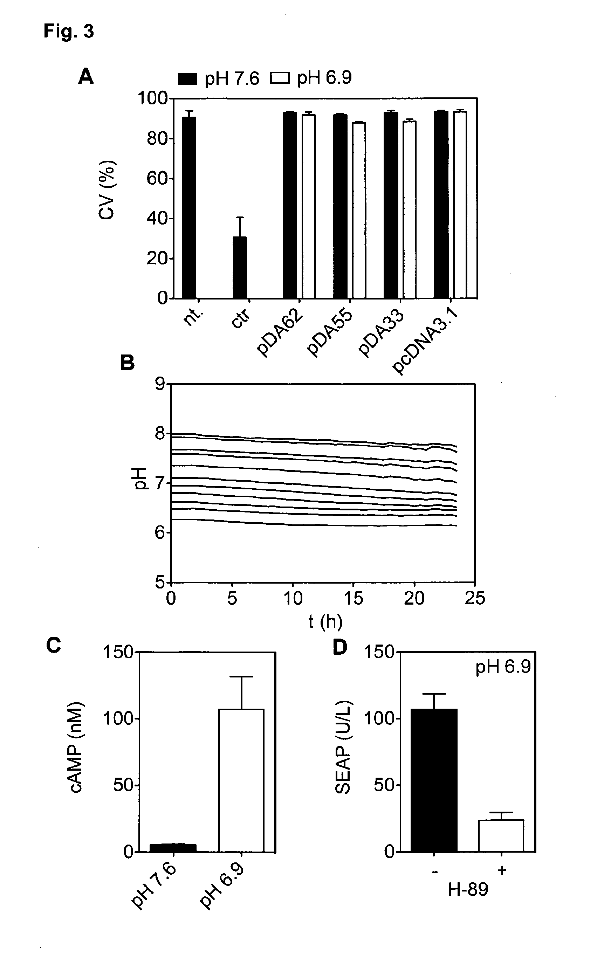

_Figure 3: pH sensor control experiments._

(A) Impact of constitutive TDAG8 expression on cell viability (CV). HEK-293T cells were cotransfected with indicated expression vectors and grown in cell culture medium adjusted to high (7.6) and low (6.9) pH. After 24 h the cells were co-stained with annexin V-FITC and Live/Dead far red cell stain and analyzed by flow cytometry. Non-staining cells were considered viable. Apoptosis-inducing rotenone (100 $\mu$M) was used to induce cell death (ctr).

(B) Continuous non-invasive real-time pH profiling of cell cultures ($1x10^5$ HEK-293T) adjusted to differerent starting pH by HCl and maintained for 24 h in HydroDishes incubated at 37°C in a standard incubator with a humidified atmosphere containing 5% $CO_2$.

(C) pH-triggered intracellular cAMP surge in pH sensor cotransfected HEK-293T (pDA62/pCK53).

(D) pH sensor based signaling cascades includes protein kinase A (PKA). HEK-293T cotransfected with pDA62 and pCK53 were treated for 1 h with the PKA inhibitor H-89 (10 $\mu$M), grown for 2 h at pH 6.9 and then shifted to pH 7.6 before SEAP levels were quantified after 24 h.

_Figure 4: pH sensor based EYFP induction kinetics._

HEK293-T cells were (co-)transfected either with pCK91 alone or in combination with pDA62, incubated in culture medium adjusted to high (7.6) and low (6.9) pH and EYFP expression was monitored using time-lapse microscopy.

_Figure 5: CO$_2$ mediated transgene induction characteristics_

(A) Henderson-Hasselbalch equation-based contour plot illustrating the correlating impact of bicarbonate and carbone dioxide ($CO_2$) on the cell culture pH.

**(B)** Experimental validation of predicted pH values shown in (A). Standard cell culture medium containing 2 g/L (_FreeStyle™_ 293 Expression Medium) and 3.7 g/L (DMEM) bicarbonate by default was maintained in normal cell culture incubators set to different percent $CO_2$ content of the atmosphere inside and the resulting culture pH was assessed using SensorDish technology.

**(C)** Simulation of $CO_2$ mediated transgene induction (CMTI). Contour plot showing predictions of percent $CO_2$ mediated transgene induction in response to bicarbonate and gaseous $CO_2$.

**(D, E)** Experimental validation of predicted $CO_2$ performance. pH sensor transgenic (pDA62/pCK53) HEK-293T cells were exposed to varying $CO_2$ **(D)** and bicarbonate **(E)** concentrations and SEAP production was profiled in the culture supernatant after 24 h.

**(F)** Dry ice-based gaseous $CO_2$-triggered induction of the pH sensor device. $5x10^4$ pH sensor transgenic (pDA62/pCK53) HEK-293T seeded per well of a 96-well plate were exposed to $CO_2$-sublimating dry ice (1 g) placed in the four central wells, and SEAP levels were assessed in the culture supernatants after 24 h. A non-induced dry-ice free setting was used as control (black).

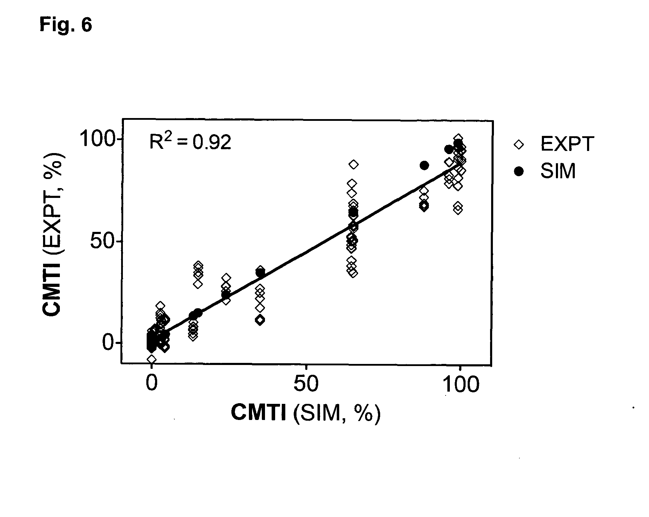

_Figure 6: CO$_2$ mediated transgene induction (CMTI) - correlation between measured and simulated values._

Correlation between simulated (SIM, black dots) and measured (EXPT, white diamants) percent $CO_2$ mediated SEAP induction. Experimental data were generated by cultivating pH sensor transgenic (pDA62/pCK53) HEK-293T cells for 24 h in medium adjusted to different bicarbonate and $CO_2$ concentrations before SEAP production was profiled in the culture supernatant. The same bicarbonate and $CO_2$ concentrations were inserted into equation (2) to simulate percent $CO_2$ mediated transgene induction (CMTI). The black line shows the linear regression of experimental data.

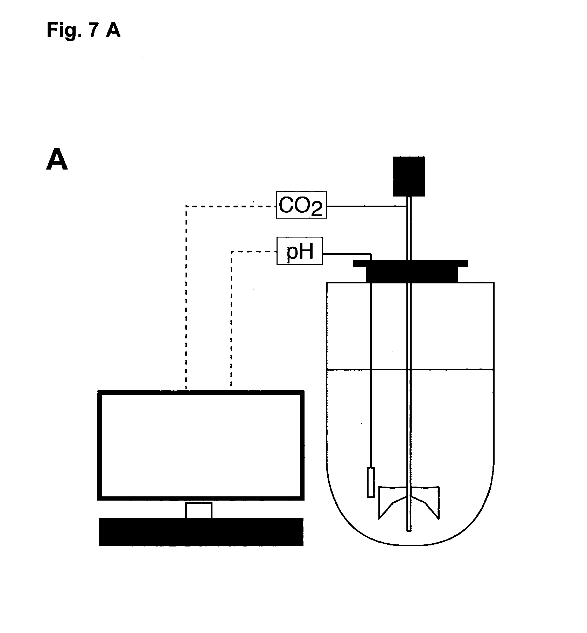

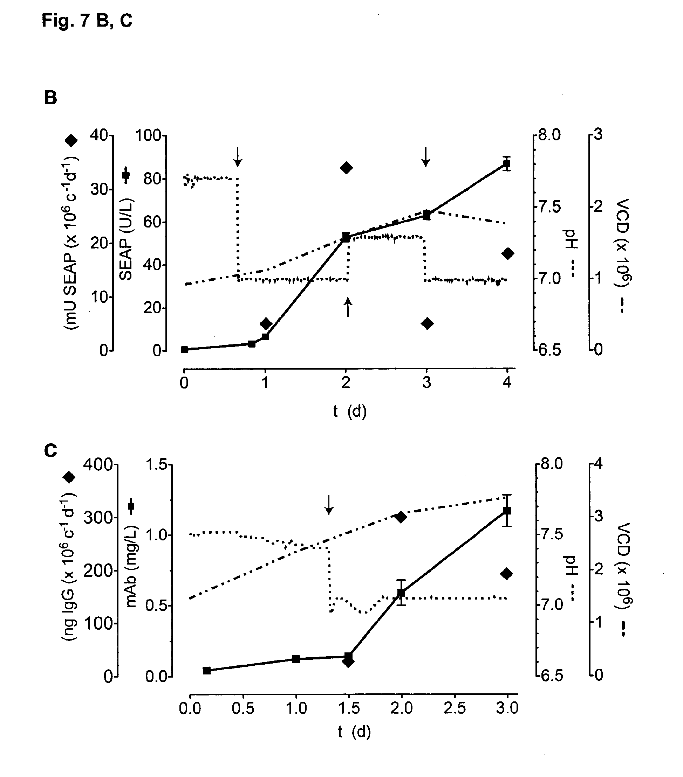

*Figure 7: Performance of CO$_2$ mediated product* gene *expression in stirred-tank bioreactors*

**(A)** Scheme of the stirred-tank bioreactor used for CO$_2$ driven product gene expression.
**(B)** CO$_2$-programmable SEAP expression in a standard stirred-tank bioreactor. pH sensor transgenic (pDA62/pCK53) *Freestyle™*-293F suspension cells were cultivated in 1 L protein-free chemically-defined medium using a stirred-tank bioreactor and SEAP production was programmed by shifts in gaseous CO$_2$ influx (arrows).
**(C)** CO$_2$ programmed production of the block-buster anti-cancer drug Rituximab. *Freestyle™*-293F suspension cells transgenic for pH sensor driven Rituximab production (pDA62/pDA146) were cultivated in 1.4 L protein-free chemically-defined medium using a stirred-tank bioreactor, and Rituximab production was induced by a shift in gaseous CO$_2$ influx (arrow).

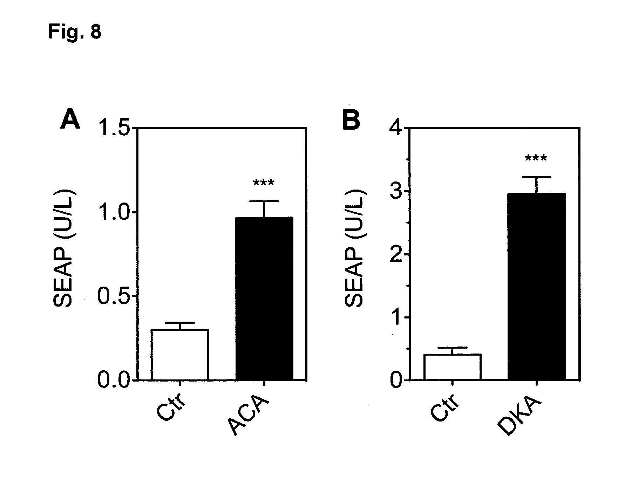

*Figure 8:* In vivo *validation of* the *pH-Sensor device*

**(A)** Wild type mice implanted with pH sensor transgenic pDA62-/pCK53-engineered HEK-293T cells (HEK$_{pH-Sensor}$, $2\times10^6$ cells; 200 cells/capsule) were treated with acetazolamide (Diamox®; ACA) to induce a metabolic acidosis, and blood SEAP levels were assessed after 24 h. Control mice received injections of PBS.
**(B)** Mice suffering from diabetic ketoacidosis (DKA) were implanted with HEK$_{pH-Sensor}$ cells ($2\times10^6$ cells; 200 cells/capsule) and blood SEAP levels were monitored 24 h after treatment. Wild-type mice receiving PBS injections served as negative control. (Data presented are mean $\pm$ SEM, n $\geq$ 10 mice, statistics by two-tailed t-test, *** $P$ <0.0001).

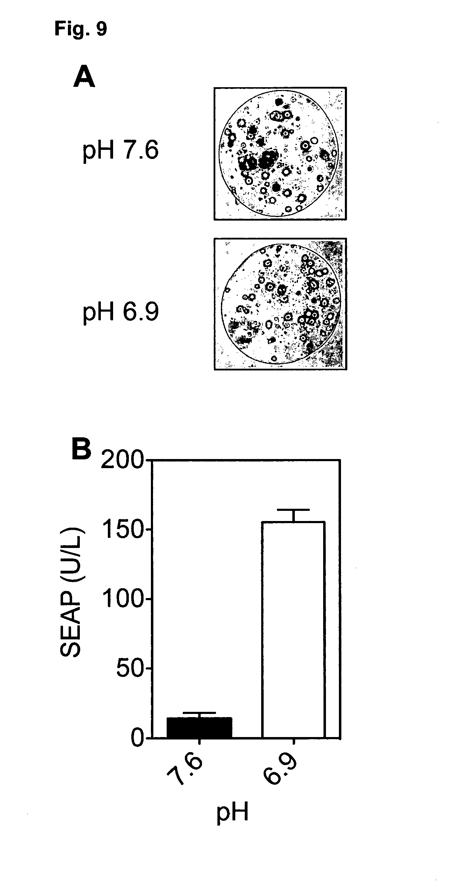

*Figure 9*: *Microencapsulation of pH sensor transgenic* cells

**(A)** Brightfield micrographs of alginate-PLL-alginate capsules containing pH sensor transgenic (pDA62/pCK53) HEK-293T (200 cells/capsule) incubated for 24 h in cell culture medium adjusted to high (7.6) and low (6.9) pH.
**(B)** SEAP production of microencapsulated cells shown in (A) (15'000 capsules).

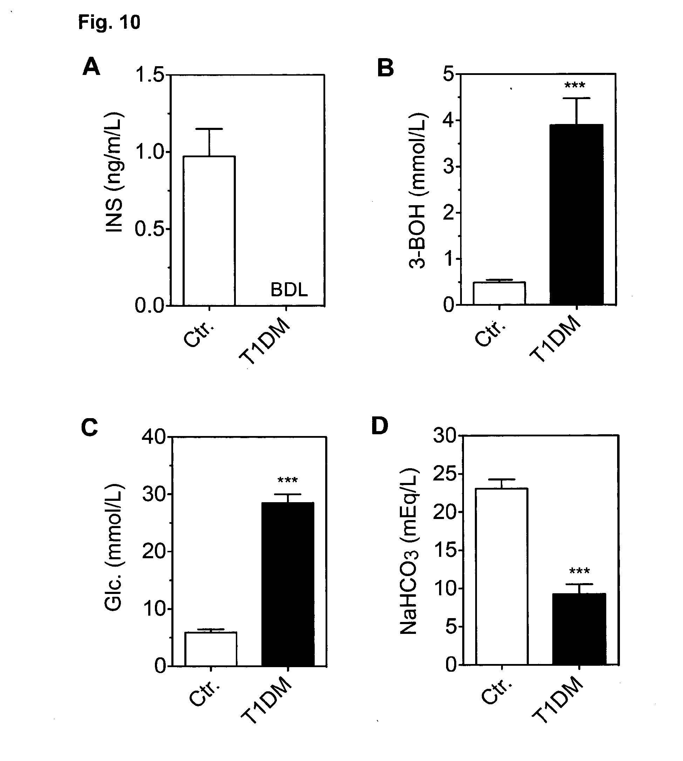

*Figure 10: Characterization of T1DM mice*

Healthy and T1 DM mice were monitored for blood **(A)** insulin, **(B)** 3-hydroxybutyrate, **(C)** glucose and **(D)** bicarbonate levels 72 h (48 h for glucose and 3-hydroxybutyrate) after alloxan (T1 DM mice) or PBS (healthy mice) injection. Insulin levels in untreated T1 DM mice were below detection limit (BDL; <0.1 ng/mL). (Data presented are mean $\pm$ SEM, n $\geq$ 10 mice, statistics by two-tailed t-test, *** $P$ <0.0001).

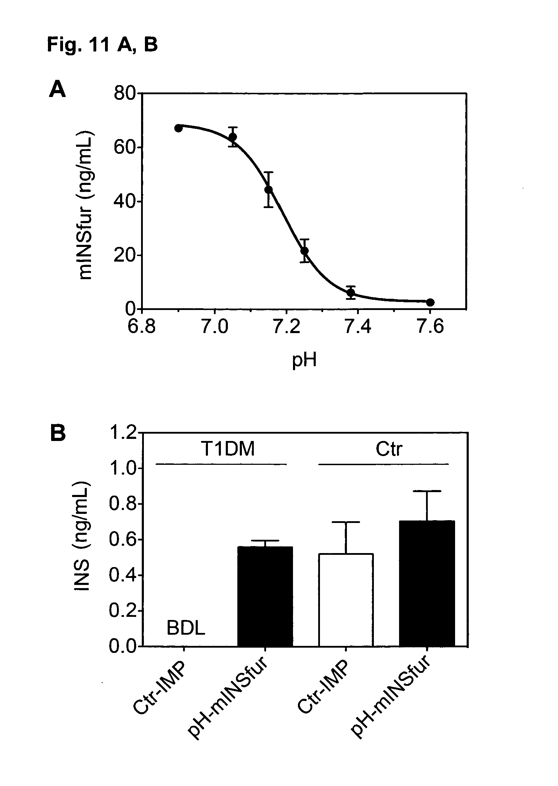

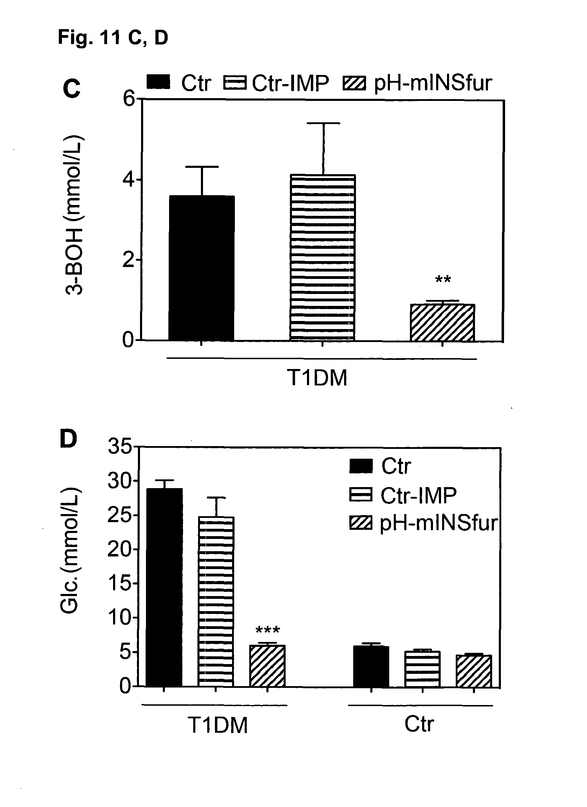

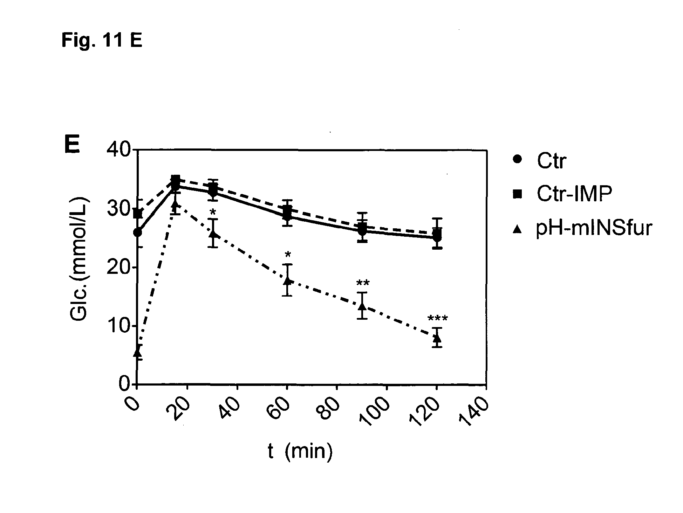

*Figure 11: Design, characterization and* in vivo *validation of a safeguard device for the treatment of diabetic ketoacidosis and type 1 diabetes mellitus (T1DM).*

(A) pH safeguard performance in cell culture. pH safeguard transgenic pDA62-/pDA145-engineered HEK-293T cells (HEK$_{pH-Guard}$) were grown for 24 h in cell culture medium adjusted to different pH before insulin production (mINSfur) was profiled in the culture supernatant. pH 7.35-7.45 represents the narrow range of human blood pH, while pH values associated with human acidosis are below pH 7.35. EC$_{50}$ = 7.18 $\pm$ 0.04.
**(B)** pH safeguard performance in mice. Mice suffering from type 1 diabetes mellitus (T1DM) were implanted with HEK$_{pH-Guard}$ and blood insulin levels (INS) were profiled after 24 h. T1 DM mice receiving placebo implants and healthy animals treated with placebo and pH safeguard implants (pH-mINSfur) served as controls (Ctr). Insulin levels in placebo-implanted T1 DM mice were below detection limit (BDL; <0.1 ng/mL).
**(C)** pH safeguard based treatment of diabetic ketoacidosis in mice. Blood 3-hydroxybutyrate (3-BOH) levels were quantified in T1 DM mice 24 h after treatment with pH safeguard implants (pH-mINSfur). Untreated and placebo-implanted T1DM animals served as controls.
**(D)** pH safeguard based control of glucose homeostasis in T1DM mice. Blood glucose (Glc) levels were quantified in T1 DM mice 24 h after treatment with pH safeguard implants. Untreated and placebo-implanted T1 DM mice as well as healthy animals receiving placebo and pH safeguard implants served as controls.
**(E)** Impact of pH safeguard implants on glucose tolerance. T1 DM mice treated with pH safeguard (pH-mINSfur), placebo (Ctr-IMP) or no implants (Ctr) received intraperitoneal glucose injections and their subsequent glycemic excursions were recorded. pH safeguard implants consist of microencapsulated pH safeguard transgenic cells ($4\times10^6$ HEK$_{pH-Guard}$ cells; 200 cells/capsule) and placebo implants contain microencapsulated pH sensor transgenic cells ($4\times10^6$ HEK$_{pH-Sensor}$; 200 cells/capsule) isogenic to HEK$_{pH-Guard}$; Data presented are mean $\pm$ SEM, n = 10 mice, statistics by two-tailed t-test, * P <0.05, ** $P$ <0.01, *** $P$ <0.0001).

**Detailed description of the invention**

The invention relates to a mammalian cell comprising

(a) a vector expressing a pH sensing receptor comprising a promoter and a polynucleotide coding for a G protein-coupled receptor, for example TDAG8, OGR1 and/or GPR4 and related receptors;
(b) a vector comprising a synthetic promoter specifically activated by said pH sensing receptor and, for example, cAMP-response elements (CRE), nuclear factor of activated T cells (NFAT) response elements, serum response elements (SRE) or 12-O-tetradecanoyl-phorbol-13-acetate response elements (TRE) fused to a polynucleotide coding for an endogenous or exogenous protein.

A "related receptor" is a receptor which is, for example, derived from the mentioned receptors TDAG8, OGR1 or GPR4. By "derived from" a receptor, is meant, in this context, that the amino acid sequence is almost identical to the amino acid sequence of the natural receptor, contains only conservative amino substitutions and remains at least 70%, preferably 80%, and more preferably 90% identical at the amino acid level. By "related to" a natural receptor such as TDAG8, OGR1 and GPR4 is meant, for purposes of the invention, that the polynucleotide sequence that encodes the amino acid sequence hybridizes to a naturally occurring polynucleotide sequence encoding receptor TDAG8, OGR1 or GPR4 under at least low stringency conditions, more preferably moderate stringency conditions, and most preferably high stringency conditions, and retains the pH sensing properties. Conservative substitution is known in the art and described by Dayhof, M.D., 1978, Nat. Biomed. Res. Found., Washington, D.C., Vol. 5, Sup. 3, among others. Genetically encoded amino acids are generally divided into four groups: (1) acidic = aspartate, glutamate; (2) basic = lysine, arginine, histidine; (3) non-polar = alanine, valine, leucine, isoleucine, proline, phenylalanine, methionine, tryptophan; and (4) uncharged polar = glycine, asparagine, glutamine, cysteine, serine, threonine, tyrosine. Phenylalanine, tryptophan and tyrosine are also jointly classified as aromatic amino acids. A substitution of one amino acid in a particular group with another amino acid in the same group is generally regarded as a conservative substitution. It is understood, for the purpose of this invention, that "derived from" and "related to" also includes compounds at the polynucleotide level comprising triplet codons coding for the same amino acid but being especially adapted to the intended host cell, e.g. mammalian cell. Such polynucleotides especially adapted to mammalian cells are particularly preferred.

A "related" receptor according to the invention is also a TDAG8, OGR1 and/or GPR4 receptor derived from a non-human animal, for example from mouse or rat.

Endogenous or exogenous proteins considered for the purpose of the invention are, for example, drug proteins, e.g., carbonic anhydrases, insulin, leptin, glucagon-like peptide 1 (glp-1), glucagon, and detoxifying enzymes, and therapeutic antibodies, such as rituximab, trastuzumab, alemtuzumab, cetuximab, bevacizumab, panitumumab, ofatumumab, etanercebt, canakinumab, and abatacept. In particular the protein is insulin with an engineered furin-cleavage site between the B and C chain.

Another class of proteins are easily detectable proteins, for example, human placental secreted alkaline phosphatase (SEAP), a fluorescent or enhanced fluorescent protein (e.g. GFP, RFP, YFP, and the like), secreted alpha amylase (SAMY), luciferase, beta-galactosidase, beta-lactamase, and glucoronidase.

In a vector expressing a pH sensing receptor comprising a G protein-coupled receptor TDAG8, OGR1 and/or GPR4 and related receptors, a promoter considered is, for example, the constitutive simian virus 40 promoter ($P_{SV40}$), the minimal human cytomegalovirus immediate early promoter ($P_{hCMVmin}$), the constitutive human cytomegalovirus promoter ($P_{hCMV}$), the human elongation factor 1$\alpha$ promoter ($P_{hEF1\alpha}$), the phosphoglycerate kinase promoter ($P_{PGK}$), the human ubiquitin promoter ($P_{hUBC}$), or the beta-actin promoter.

The invention further relates to a mammalian cell as defined above cultured in any kind of bioreactor, e.g. in standard bioreactors, roller bottles or wavebags.

Furthermore the invention relates to the carbon dioxide-inducible production of polypeptides, e.g. monoclonal antibodies or insulin, in bioreactors as defined above.

In particular the invention relates to a bioprocessing method for the $CO_2$-inducible production of biopharmaceuticals wherein a mammalian cell of the invention expressing the biopharmaceutical is cultured in a bioreactor with pH control by introducing $CO_2$. The mammalian cell is cultured under standard conditions, preferably using media and nutritients particularly adapted to the mammalian cell. The pH of the cell mass is monitored using a pH electrode. Suitable software is used to control the product expression through the pH using gaseous $CO_2$ to acidify and lower the pH, or aqueous NaOH to raise the pH.

The invention further relates to a mammalian cell as defined above in a nanocontainer or microcontainer, e.g. in encapsulated form. A nanocontainer may be a virus, preferably an attenuated virus, in particular a viral capsid, synthetic or semi-synthetic nano- or microparticles, such as spheres or tubes of a suitable geometry to incorporate mammalian cells, and the nano- or microcontainers formed *in situ* by encapsulation of mammalian cells, for example with alginate-poly-L-lysine. A particular example of a suitable nano- or microcontainer is the hollow fibre manufactured under the trade name CELLMAX.

The invention further relates to a mammal excluding man comprising a mammalian cell as described, in particular a mammalian cell in a nano- or microcontainer.

Furthermore the invention relates to a treatment of acidosis-related disorders and type 1 diabetes, comprising administering to a patient in need thereof an effective amount of a mammalian cell according to the invention, preferably in encapsulated form.

Acidosis-related disorders are, in particular, metabolic acidosis such as diabetic ketoacidosis, alcoholic ketoacidosis, lactic acidosis, drug intoxications (from ethylene glycol, methanol, paraldehyde, carbonic anhydrase inhibitors, aspirin), renal tubular acidosis or any kind of respiratory acidosis.

In particular the invention relates to a mammalian cell wherein the promoter functionally connected to the G protein-coupled receptors TDAG8, OGR1 or GPR4 is selected from the constitutive simian virus 40 promoter ($P_{SV40}$), the minimal human cytomegalovirus immediate early promoter ($P_{hCMV}$), and the human elongation factor 1$\alpha$ promoter ($P_{hEF1\alpha}$).

In a further particular embodiment the vector comprising a synthetic promoter ($P_{CRE}$) containing cAMP-response elements (CRE) is fused to a bicistronic unit comprising polynucleotides coding for heavy and light chain of a therapeutic antibody, in particular of rituximab.

In another particular embodiment the vector comprising a synthetic promoter ($P_{CRE}$) containing cAMP-response elements (CRE) is fused to a polynucleotide coding for insulin or pro-insulin, in particular a pro-insulin containing a furin cleavage site between B chain and C peptide.

Capitalizing on ectopic expression of TDAG8 a designer cascade was assembled that was able to precisely sense culture pH (pH sensor), trigger transgene expression in mammalian cells grown in culture and state-of-the-art bioreactors by gaseous $CO_2$ ($CO_2$ control) as well as correct diabetic ketoacidosis and restore glucose homeostasis in mice suffering from type 1 diabetes (pH safeguard). Synthetic pH sensor, $CO_2$ control and pH safeguard may significantly advance the production of difficult-to-express protein therapeutics as well as gene and cell-based therapies.

*Design and validation of a synthetic mammalian pH sensor.*

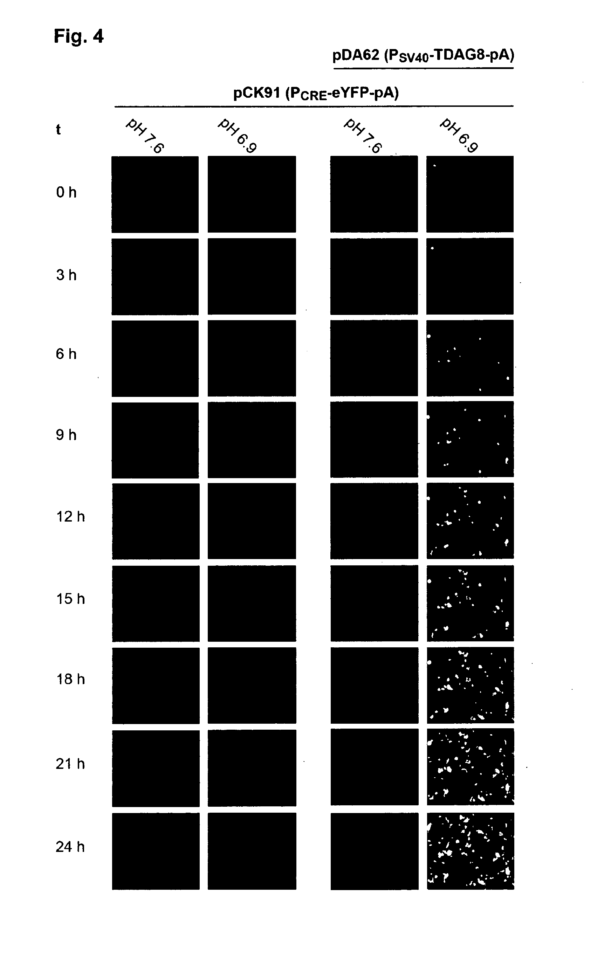

Although the precise physiologic function of the G protein-coupled receptor TDAG8 remains elusive it has been shown to sense extracellular protons and trigger a heterotrimeric $G_s$-protein response that leads to activation of the adenylate cyclase and results in an intracellular cyclic AMP (cAMP) surge (Ishii S. et al., 2005, J Biol Chem 280, 9083-9087). By functionally linking cAMP levels to activation of the protein kinase A (PKA), phosphorylation of the cAMP-responsive binding protein 1 (CREB1) and CREB1-mediated induction of synthetic $P_{CRE}$ promoters, extracellular pH could be directly rewired to desired transgene expression (Fig. 1). Indeed when cotransfecting TDAG8 expression units driven by different constitutive promoters ($P_{hEF1\alpha}/P_{hCMV}/P_{SV40}$-TDAG8-pA; pDA55/pDA33/pDA62) with $P_{CRE}$-driven SEAP ($P_{CRE}$-SEAP-pA, pCK53) or EYFP ($P_{CRE}$-EYFP-pA, pCK91) expression vectors into human embryonic kidney cells (HEK-293T), a HEK-293-derived production cell line adapted for growth in protein-free suspension cultures (*Freestyle*™ 293-F), Chinese hamster ovary cells (CHO-K1), and human mesenchymal stem cells (hMSC-TERT), reporter gene expression could be reliably switched ON and OFF in all cell lines by corresponding low and high pH within the human physiologic range (pH6.9 and pH7.6) (Fig. 2A). As expected when rewiring TDAG8 to $P_{CRE}$ *via* a compatible endogenous signaling cascade, the pH sensor showed cell type-specific performance variations (Fig. 2A). A comprehensive set of control experiments showed that (i) ectopic expression of pTDAG8 did neither reduce cell viability nor maximum SEAP production levels over the entire pH range (Figs. 2B and 3A), (ii) that the culture pH could be precisely adjusted and remained constant during expression profiling (Fig. 3B), (iii) that $P_{CRE}$-driven SEAP and EYFP production was completely repressed in the absence of TDAG8 expression within the entire physiologic pH range (Figs. 2B and 4), (iv) that pH-triggered expression switches correlated with cAMP levels (Fig. 3C) and (v) that the TDAG8-to-$P_{CRE}$ signaling could be interrupted by the PKA inhibitor H-89 confirming the exclusive rewiring (Fig. 3D).

The pH sensor device was completely shut down within the normal physiologic pH range of the human body (pH 7.35-7.45) and was gradually induced at lower pH (pH 7.35 - pH 6.5) that correlated with pathologic acidosis typically associated with certain drug treatment (Hafner P. et al., 2008, Am J Physiol-Cell Ph 295, C1658-C1667), alcohol intoxication (Brent J., 2009, N Engl J Med 360, 2216-2223) and diabetic ketoacidosis (Barker J.M. et al., 2004, Diabetes Care 27, 1399-1404) (Fig. 2B; $EC_{50}$ = pH 7.15 $\pm$ 0.03). The pH-dependent transgene expression switches were reversible (Fig. 2C), showed rapid induction kinetics with robust expression levels (Fig. 2D) and reached maximum transgene expression levels only when continuously induced for over 2.5 h suggesting that the pH sensor was buffered against short transient physiologic pH variations (Fig. 2E). While the pH sensor was able to reliably detect the current culture pH with high precision it could also be used to tune desired transgene expression by addition of different acids including 3-hydroxybutyric acid, one of the key organic acids responsible for diabetic ketoacidosis (Fig. 2F). Overall, the pH sensor provides a novel technology for trigger-inducible product gene expression and shows all critical performance characteristics that are required to serve as a physiologic acidosis sensor device.

*Carbon dioxide-inducible transgene control*

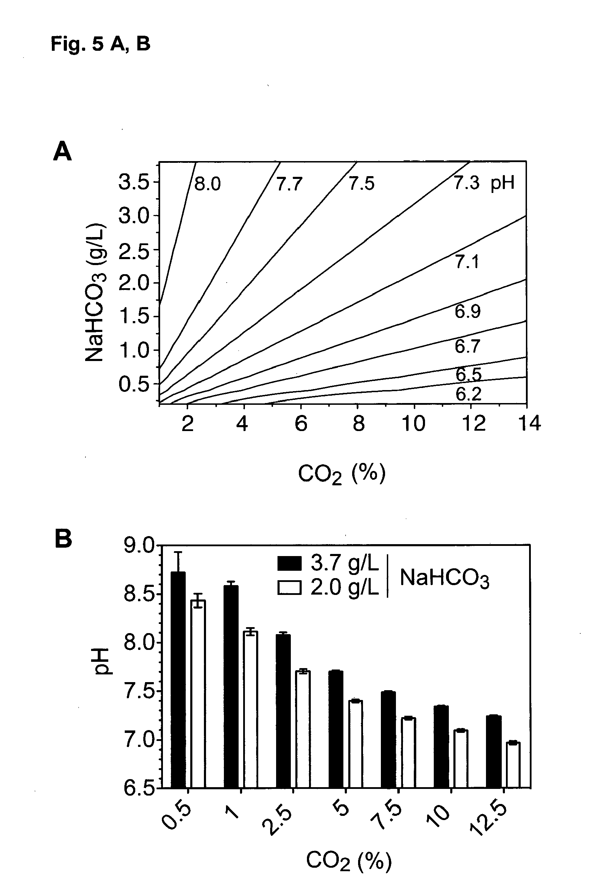

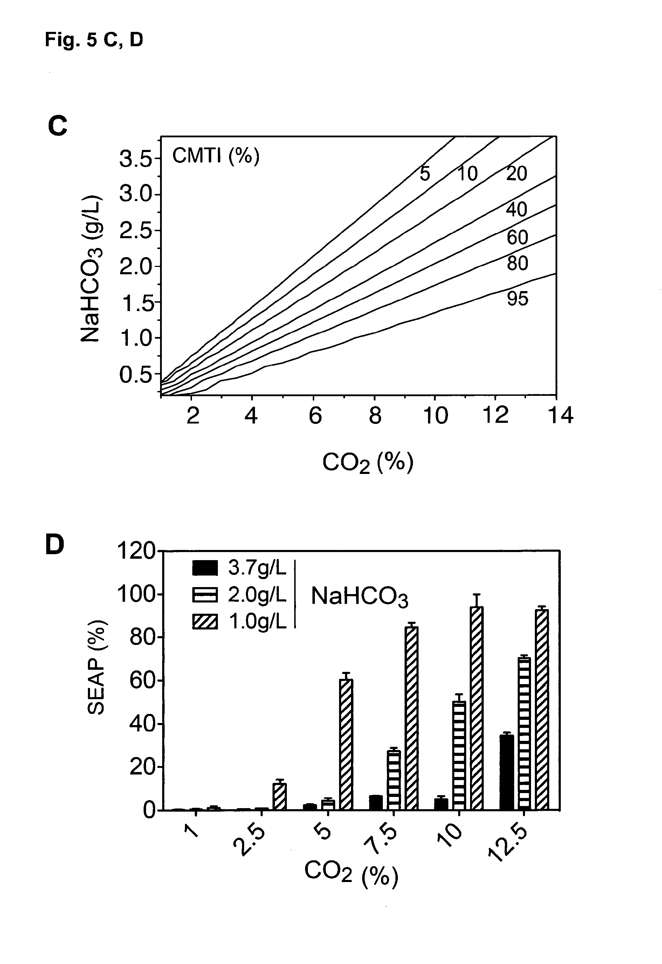

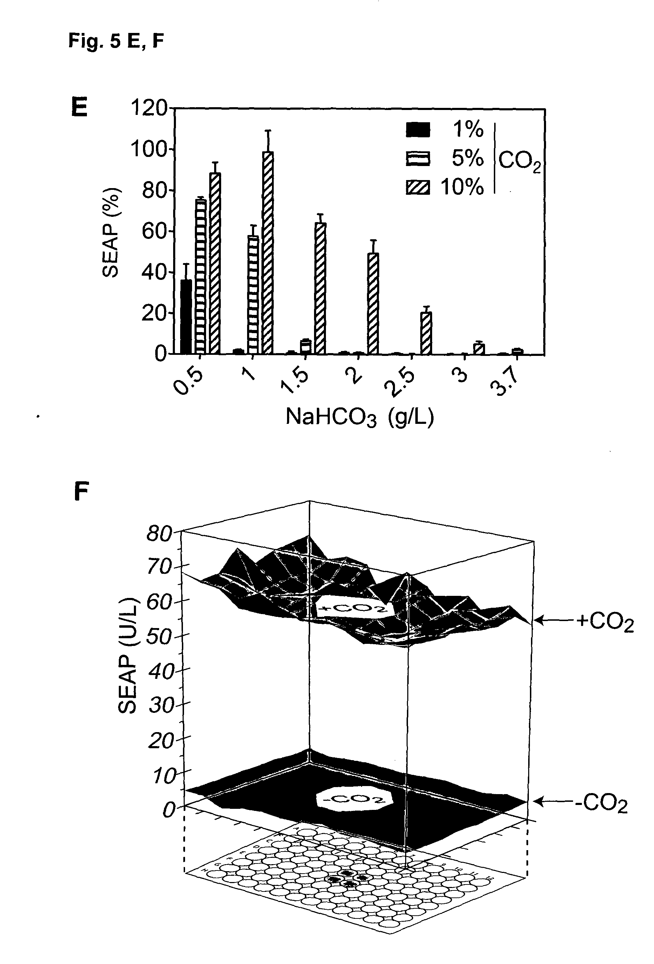

Industrial bioprocesses tailored to biopharmaceutical manufacturing of protein therapeutics capitalize on the $CO_2$-bicarbonate buffering system to control pH in bioreactors by gaseous $CO_2$. Using a Henderson-Hasselbalch equation-based contour plot correlating percent $CO_2$ and bicarbonate concentration with pH of culture medium, the culture pH resulting from a specific combination of bicarbonate concentration and percent $CO_2$ could be precisely calculated (Fig. 5A). When exposing *FreeStyle™* 293 Expression Medium (2 g/L bicarbonate) and DMEM (3.7 g/L bicarbonate) containing fixed bicarbonate levels to different concentrations of gaseous $CO_2$ the resulting pH, which was profiled in culture medium in real time using a SensorDish Reader, highly correlated with the data shown in the contour plot (Fig. 5A,B). This allows to precisely tune cell culture pH by gaseous $CO_2$. A mathematical model coordinated to the system parameters enabled simulation of $CO_2$ driven transgene induction in response to any given combination of bicarbonate concentration and gaseous $CO_2$ (Fig. 5C). In particular, the model predicts that system mediated transgene transcription positively correlates with increasing $CO_2$ levels while the bicarbonate concentration determines the amount of $CO_2$ required for maximum expression levels (Fig. 5C). $CO_2$ mediated SEAP expression of pH sensor transgenic HEK-293T (HEK$_{pH-Sensor}$, pDA62/pCK53) correlated with model predictions ($R^2$= 0.92, P<0.0001) and confirmed bicarbonate-determined $CO_2$-triggered maximum SEAP production levels (Fig. 6). Overall, the system enabled precise programmable, adjustable and traceless gas-inducible transgene expression in mammalian cells (Fig. 5D,E). While most users may choose to adjust the $CO_2$ levels of their incubators to fine-tune desired transgene expression, gas-inducible transcription control could also be confirmed by placing dry-ice pellets into the four central wells of a 96-well plate containing HEK$_{pH-Sensor}$ in all remaining wells (Fig. 5F). $CO_2$ rapidly sublimates from the dry-ice pellets and re-dissolves into neighboring wells thereby acidifying the culture medium and boosting SEAP production (Fig. 5F).

*$CO_2$ based production of Rituximab in protein-free suspension cultures grown in stirred-tank bioreactors*

State-of-the-art bioreactors in the industry contain a pH electrode that monitors proton concentration of the production culture and feedback controls base and gaseous $CO_2$ influx to maintain pH at preset values (Fig. 7A). Taking advantage of a bioreactor standard pH-control hardware and electronics to remotely control and program product gene, expression to desired levels was accomplished with gaseous $CO_2$. In a prototype biopharmaceutical manufacturing scenario pH sensor transgenic (pDA55/pCK53) SEAP-producing *Freestyle™*-293F suspension cells was cultivated in protein-free chemically-defined culture medium using a reference stirred-tank bioreactor operated in batch mode. $CO_2$ influx or base pump activity were programmed to increase or decrease at specific time points, which resulted in pre-defined pH changes of the production medium and adjusted SEAP expression of pH sensor transgenic production cells (Fig. 7B). Importantly, the $CO_2$-triggered pH changes in the production medium highly correlated with changes in specific SEAP productivity, which confirms the reversibility, robustness and precision of the $CO_2$ modulated pH sensor device in a proof-of-concept biopharmaceutical manufacturing setting (Fig. 7B).

The $CO_2$ triggered bioreactor-based manufacturing of protein pharmaceuticals was extended to the production of Rituximab, a chimeric monoclonal anti-CD20 immunoglobulin (IgG) that is clinically licensed for the treatment of diseases characterized by excessive, overactive or dysfunctional B cells such as lymphomas, leukemias, transplant rejection and certain types of autoimmune disorders (Cheson B.D. and Leonard J.P., 2008, N Engl J Med 359, 613-626). Using *Freestyle™*-293F suspension cells engineered for constitutive TDAG8 expression (pDA55) and P$_{CRE}$-driven bicistronic expression of Rituximab's heavy and light chains (pDA146) it was possible to precisely induce production of this blockbuster drug after an expansion phase of 36 h by shifting the culture pH from 7.45 to 7 using ph sensor technology (Fig. 7C). As a unique gas-inducible transcription-control modality the $CO_2$ control system uses a most natural, industry-approved, FDA-licensed and traceless trigger compound to precisely time and remote-control product gene expression in bioreactors without creating any downstream processing, validation or approval challenges.

*A prosthetic network correcting diabetic ketoacidosis and restoring glucose homeostasis in type 1 diabetes*

The $CO_2$-bicarbonate buffering system keeps normal blood and tissue pH within a very narrow range (pH 7.35-7.45). A tissue pH below 7.35 is known as acidosis, a medical condition that is difficult to diagnose due to its wide range of symptoms and diverse pathophysiologies. To validate *in vivo* performance and precision of the pH sensor that is able to discern normal (>7.35) and pathologic (<7.35) pH in mammalian cell cultures (Fig. 2B), HEK$_{pH-Sensor}$ was implanted into wild type and acidotic mice. Treatment of animals for five days with the carbonic anhydrase inhibitor acetazolamide (ACA), a clinically licensed drug (Diamox®) for the treatment of glaucoma, epileptic seizures and intercranial hypertension, has been shown to induce metabolic acidosis. Because blood SEAP levels were significantly increased in acidotic mice compared to wild type animals, pH sensor implants were able to tap into the animals' circulation, monitor proton levels, process pH information and produce a tuned transgene expression response which resulted in SEAP secretion and accumulation in the bloodstream (Fig. 8A). *In vitro* control experiments confirmed that the capsule structure was inert to low pH and facilitated diffusion of SEAP produced by encapsulated cells in response to low pH (Fig. 9A,B).

With the pH sensor precision validated in mice, a prosthetic network for the treatment of diabetic ketoacidosis and type 1 diabetes was designed. Insufficiently treated type 1 diabetes leads to a life-threatening medical condition known as diabetic ketoacidosis that is characterized by critically low blood pH values resulting from excessive production of ketones (acetoacetic acid, β-hydroxybutyrate) by uncontrolled fatty acid metabolism in the absence of ketosis-inhibiting insulin. By functionally linking the pH sensor (sensor device: pDA62) to expression of secretion-engineered furin-cleavable proinsulin 1 (effector device: pDA145; $P_{CRE}$-mIns-pA), enabling efficient insulin maturation and secretion in non-beta cells (Hay C.W. and Docherty K., 2003, J Mol Endocrinol 31, 597-607), a prosthetic pH safeguard network was created. Corresponding transgenic cells (HEK$_{pH-Guard}$) were set to sense diabetic ketoacidosis *via* low pH and produce a tuned insulin secretion response that reduces ketosis, restores physiologic pH and controls glucose homeostasis.

Chemically-induced type 1 diabetic mice showed undetectable insulin levels (Fig. 10A), high 3-hydroxybutyrate levels (Fig. 10B), hyperglycemia (Fig. 10C) and low bicarbonate concentrations (Fig. 10D) in the blood as well as high glucose (>55 mmol/L) and acetoacetic acid (>15 mmol/L) concentrations in the urine. When implanted HEK$_{pH-Sensor}$ the animals showed increased SEAP serum levels, confirming that they were indeed suffering from diabetic ketoacidosis (Fig. 8B). Also, pre-validation of the pH safeguard system in cell culture confirmed that HEK$_{pH-Guard}$ secretes high doses of insulin at acidosis relevant pH (Fig. 11A; $EC_{50}$ = pH 7.18 ± 0.04). Upon implantation of microencapsulated batches of the same HEK$_{pH-Guard}$ cell population into mice suffering from diabetic ketoacidosis, HEK$_{pH-Guard}$ rapidly detected and scored pH and produced an efficient systemic insulin response with insulin levels matching those of healthy control mice which have their insulin production tuned by the native pancreatic beta-cell population (Fig. 11B). As a consequence of systemic insulin production, ketosis was reduced and 3-hydroxybutyrate levels decreased significantly (Fig. 11C). Also, while untreated mice suffering from type 1 diabetes showed dramatic hyperglycaemia, animals with the inventive implants had their blood glucose levels corrected to concentrations found in healthy control mice (Fig. 11D). The system also protected mice from glycaemic excursions in glucose tolerance tests confirming that the insulin-based effector device was fully functional and efficiently restored glucose homeostasis in treated animals (Fig. 11E).

*Discussion*

Trigger-inducible transcription control of heterologous transgenes has become standard practice in gene-function analysis, functional genomic research, drug discovery, biopharmaceutical manufacturing of difficult-to-produce protein therapeutics and drug targets, tissue engineering, and prototype gene and cell-based therapies. After two decades of constant evolution of the inaugural Tet-based transcription control design most available control switches show improved low-leaky and maximum expression levels, are completely adjustable and reversible and exist in the preferred ON-type configuration which induces expression in the presence of the trigger compound. However, assembling synthetic transcription-control switches responsive to heterologous inducer compounds or endogenous trigger metabolites that may be licensed for biopharmaceutical manufacturing of protein therapeutics, used to remote-control therapeutic transgenes or plugged into host physiologic networks to sense and correct metabolic disorders in future gene and cell-based therapies remains a current priority. Equally challenging is the design of synthetic transcription-control modalities with an inducible compound level that precisely matches the physiologic or clinically relevant concentration range. The latest generation of synthetic transcription controllers are fine-tuned by licensed food additives (vanillic acid, 2-phenylethyl butyrate) and vitamins (vitamin B7), cosmetics ingredients (phloretin), amino acids (L-tryptophan, L-arginine), hormones (luteinizing hormone) or pathologic concentrations of disease metabolites such as uric acid. With the design of signaling cascades functionally linking extracellular trigger sensing by cell-surface receptors to activation of chimeric promoters transcription control has entered a new era, which is characterized by using heterologous component- and cofactor-free all-human control devices that fine-tune target gene expression in response to extracellular input with unmatched reliability, sensitivity and precision (Ye H. et al., 2011, Science 332, 1565-1568). During the past years, gene switch-based designer circuits have also successfully been tested in animal models of human disorders which foster advances of future treatment strategies. Pioneering examples include remote-controlled fine-tuning of transgene expression in engineered cell implants using electromagnetic waves in the visible or radio-frequency range, transdermal control by beauty creams and devices improving conception. The most recent generation of therapeutic designer circuits such as the one tailored for the treatment of gouty arthritis and the tumor lysis syndrome function as prosthetic networks. Prosthetic networks are synthetic sensor-effector gene circuits that seamlessly operate in engineered cell implants and constantly sense, monitor and score metabolic disturbances and peak-level concentrations of circulating pathologic metabolites, immediately process such signals and coordinate an adjusted therapeutic response in an automatic and self-sufficient manner.

The pH sensor device of the present invention provides a new dimension to transgene-transcription control in mammalian cells both in terms of concept and precision. The trigger compound are protons which are an intrinsic part of aqueous solutions that constitute life on this planet. The pH sensor can either be induced by increasing the amount of protons in solution by addition of (in)organic acids or by shifting the ubiquitous $CO_2$-bicarbonate buffering system to lower pH (higher hydrogen ion levels) using gaseous $CO_2$. This establishes the pH sensor device as a robust, reversible, adjustable and side effect-free gas-inducible transcription control system. Since state-of-the-art bioprocessing also takes advantage of the $CO_2$-bicarbonate buffering system to adjust pH in bioreactors, the technology of the present invention is particularly suited for biopharmaceutical manufacturing of protein therapeutics. To achieve the highest possible titers product gene expression often needs to be adjusted to optimal levels especially for difficult-to-express biopharmaceuticals which are slightly cytotoxic or impair growth, such as anti-cancer drugs.

The pH sensor shows high-level sensitivity and precision as it is functional within the entire physiologic pH range and significantly induced at pH below 7.35 which marks the transition between normal and pathologic pH. This precision has enabled the design of a closed loop prosthetic network that links pH sensor to expression of a secretion-engineered insulin variant. This application of the invention senses diabetic ketoacidosis in mice, corrects the acidotic state and restores glucose homeostasis. So far, the prevailing hypothesis was to engineer beta-cell mimetics that provide glucose-sensitive expression of insulin. However, since the design of synthetic glucose sensor circuitry matching the sensitivity of blood-glucose levels remains an ongoing challenge, alternative metabolites or metabolic states correlating with blood-glucose levels might be considered for indirect glucose sensing and coordinated insulin production. The present system represents a first example for such a closed loop prosthetic glucose homeostasis network, in which high glucose levels in type 1 diabetic mice result in the onset of ketoacidosis shifting pH below 7.35. This immediately triggers insulin production that feed-back represses ketosis and restores glucose homeostasis. In the best of synthetic biology's tradition, the pH safeguard system is a prosthetic network whose functionality does not exist in humans yet is entirely built of human components. Due to its modular design it will be straightforward to replace the sensor/effector componentry of the pH safeguard system and adapt this prosthetic network strategy to other metabolic disorders and provide novel treatment concepts for future gene- and cell-based therapies.

**Experimental Procedures**

Vector design

Comprehensive design and construction details for all expression vectors are provided in the Table.

| Plasmid | Description and Cloning Strategy | Reference or Source |
|---|---|---|
| pcDNA3.1 (+) | Mammalian expression vector ($P_{hCMV}$-MCS-pA). | Life Technologies, Carlsbad, CA, USA |
| pSEAP2-control | Constitutive SEAP expression vector ($P_{SV40}$-SEAP-pA). | Clontech, Mountain View, CA |
| pCK25 | Constitutive mUTS expression vector ($P_{hEF1\alpha}$-mUTS-pA). | Kemmer C. et al., 2010, Nat Biotechnol 28, 355-360 |
| pCK53 | $P_{CRE}$-driven SEAP expression vector ($P_{CRE}$-SEAP-pA). | Kemmer C. et al., 2011, J Control Release 150, 23-29 |
| pCK91 | $P_{CRE}$-driven EYFP expression vector ($P_{CRE}$-EYFP-pA). | Kemmer C. et al., 2011, loc. cit. |
| pTDAG8 | pCMV-SPORT6-based vector containing TDAG8 full-length cDNA (SB, cat. no. IRATp970B0554D). | GenBank: BC035633 |
| pDA139 | pUC57-derived vector containing mINS. | GenScript, Piscataway, NJ |
| pAS55 | Constitutive Rituximab expression vector ($P_{hCMV}$-$LC_R$-$IRES_{EMCV}$-$HC_R$-pA). $LC_R$ was excised from pETR1057 (Glycart) with *Pst*I/*Xho*I. $IRES_{EMCV}$ was amplified from pIRES2-EGFP (Clontech) using oligonucleotides oAS09 (5'-GAGACTCGAGGATCCGCCCCTCTCCCTC-3') and oAS10 (5'-GAGAGCGCGCGTTGTGGCCATATTATCATCGTG-3') and restricted with *Xho*I/*BssH*II. $HC_R$ was excised from pETR1119 (Glycart) with *BssH*II/*Spe*I. $LC_R$-$IRES_{EMCV}$-$HC_R$ was constructed by triple-ligation and cloned into pEGFP-N1 (Clontech) (*Pst*I/*Xba*I). |  |
| pMM28 | Constitutive SEAP expression vector ($P_{hEF1\alpha}$-SEAP-pA). $P_{hEF1\alpha}$ was excised from pCK25 by *Mlu*I/*Eco*RI and cloned into the corresponding sites (*Mlu*I/*Eco*RI) of pSEAP2-control. |  |

(continued)

| Plasmid | Description and Cloning Strategy | Reference or Source |
|---|---|---|
| pDA33 | Constitutive TDAG8 expression vector ($P_{hCMV}$-TDAG8-pA). TDAG8 was PCR-amplified from pTDAG8 using oligonucleotides ODA90 (5'-GCTG GCTAGCATGAACAGCACATGTATTGAAGAAC-3', NheI underlined) and ODA91 (5'-AGACTCGAGCTACTCAAGGACCTCTAATTC-3', XhoI underlined), restricted with NheI/XhoI and cloned into the corresponding sites (NheI/XhoI) of pcDNA3.1(+). | |
| pDA55 | Constitutive TDAG8 expression vector ($P_{hEF1\alpha}$-TDAG8-pA). TDAG8 was excised from pDA33 using NheI/XbaI and cloned into the compatible sites (SpeI/XbaI) of pMM28. | |
| pDA62 | Constitutive TDAG8 expression vector ($P_{SV40}$-TDAGB8-pA). TDAG8 was PCR-amplified from pTDAG8 using oligonucleotides ODA125 (5'-GGGTTT AAACGGGCCCTCTAGAC-3', XbaI underlined) and ODA132 (5'-ACCCG GAATTCTAGGGAGACCCAAGCTGGCTAG-3', EcoRI underlined), restricted with EcoRI/XbaI and cloned into the corresponding sites (EcoRI/XbaI) of pSEAP2-control. | |
| pDA145 | $P_{CRE}$-driven mINS expression vector ($P_{CRE}$-mINS-pA). mINS was excised from pDA139 using HindIII/XbaI and cloned into the corresponding sites (HindIII/XbaI) of pCK53. | |
| pDA146 | $P_{CRE}$-driven Rituximab expression vector ($P_{CRE}$-$LC_R$-$IRES_{EMCV}$-$HC_R$-pA). The bicistronic Rituximab-encoding unit ($LC_R$-$IRES_{EMCV}$-$HC_R$) was PCR-amplified from pAS55 using oligonucleotides ODA194 (5'-AGTTCAGAAGCTTCCAC CATGGATTTTCAGGTGCAG-3', HindIII underlined) and ODA195 (5'-GC TCTAGATCATTTACCCGGAGACAGGGAG-3', XbaI underlined), restricted with HindIII/XbaI and cloned into the corresponding sites (HindIII/XbaI) of pCK53. | |
| Abbreviations: EYFP, enhanced yellow fluorescent protein; $HC_R$, Rituximab heavy chain; $IRES_{EMCV}$, internal ribosome entry site derived from the encephalomyocarditis virus; $LC_R$, Rituximab light chain; MCS, multiple cloning site; mINS, modified rodent pro-insulin 1 containing a furin cleavage site between B chain and C peptide; mUTS, mammalian urate-dependent transsilencer; pA, polyadenylation signal; $P_{CRE}$, synthetic mammalian promoter containing a cAMP-response element; $P_{hCMV}$, human cytomegalovirus immediate early promoter; $P_{hEF1\alpha}$, human elongation factor 1 alpha promoter; $P_{SV40}$, simian virus 40 promoter; Rituximab, anti-CD20 chimeric monoclonal antibody (Rituxan®, MabThera®); SB, Source Bioscience Lifesciences, Nottingham, UK; SEAP, human placental secreted alkaline phosphatase; TDAG8, T cell death-associated gene 8 encoding GPR65). | | |

Key plasmids include: pCK53 contains a $P_{CRE}$-driven SEAP expression unit that is induced by the cAMP response element-binding protein 1 (CREB1; $P_{CRE}$-SEAP-pA). pDA55 and pDA62 encode constitutive expression of the T cell death-associated gene 8 (TDAG8) driven by the human elongation factor 1 alpha ($P_{hEF1\alpha}$-TDAG8-pA) and simian virus 40 ($P_{SV40}$-TDAG8-pA) promoters, respectively. pDA145 mediates $P_{CRE}$-driven expression of a rodent pro-insulin 1 harboring a furin cleavage site between B chain and C peptide. pDA146 is a $P_{CRE}$-driven Rituximab (Rituxan®, MabThera®) expression vector ($P_{CRE}$-$LC_R$-$IRES_{EMCV}$-$HC_R$-pA; $LC_R$/$HC_R$ Rituximab heavy and light chains, $IRES_{EMCV}$, internal ribosome entry site derived from the encephalomyocarditis virus).

Mammalian cell culture and transfection

Human embryonic kidney cells (HEK-293T, ATCC: CRL-11268) and human bone marrow stromal cells transgenic for the catalytic subunit of human telomerase (hMSC-TERT, (Simonsen, J.L. et al., 2002, Nat Biotechnol 20, 592-596)) were cultured in Dulbecco's modified Eagle's medium (DMEM, Invitrogen, Basel, Switzerland) supplemented with 10% fetal calf serum (FCS; Bioconcept, Allschwil, Switzerland; lot no. PE01026P) and 1% penicillin/streptomycin solution (Sigma-Aldrich, Munich, Germany). Chinese hamster ovary cells (CHO-K1, ATCC: CCL-61) were grown in ChoMaster® HTS (Cell Culture Technologies, Gravesano, Switzerland) supplemented with 5% FCS and 1% penicillin/streptomycin solution. Freestyle™ 293-F suspension cells (Life Technologies Corporation, Carlsbad, CA, USA, cat. no. R790-07) were cultivated in FreeStyle™ 293 Expression Medium (Life Technologies Corporation, cat. no. 12338018) supplemented with 1% penicillin/streptomycin solution and grown in shake flasks placed on an orbital shaker set to 100-150 rpm (IKA KS 260 basic, IKA-Werke GmbH, Staufen, Germany). Unless stated otherwise, all cell types were cultivated at 37°C in a humidified atmosphere containing 5% $CO_2$. Cell number and viability were quantified using an electric field multi-channel cell counting device (Casy® Cell Counter and Analyser Model TT; Roche Diagnostics GmbH, Basel, Switzerland). For transfection, a solution containing 0.5 μg plasmid DNA (1 μg for Freestyle™ 293-F) and 3 μL

PEI (1 mg/mL) was incubated in 0.2 mL FCS-free DMEM for 30 min at 22°C before it was added dropwise to $1 \times 10^5$ cells ($1 \times 10^6$ *Freestyle™* 293-F) seeded per well of a 24-well plate 24 h before transfection. Unless stated otherwise, the transfection medium was replaced by standard cultivation medium adjusted to specific pH after 7 h and transgene expression was profiled after 24 h.

pH control and profiling

For non-invasive real-time profiling of the culture pH the cells were cultivated in 24-well HydroDishes (Batch: HD-1109-01) placed on a SensorDish Reader (SDR, PreSens Precision Sensing GmbH, Regensburg, Germany) inside an incubator (HERAcell 150i, Thermo Fisher Scientific, MA, USA) set to 37°C and 5% $CO_2$. Unless stated otherwise, DMEM (10% FCS, 1% Pen/Strep) was adjusted to different physiological pH (6.0-8.0) by adding appropriate amounts of 1 N hydrochloric acid or 1 N sodium hydroxide. pH of the culture medium was validated using the HydroDish/SensorDish Reader setup.

Analytical assays

cAMP: Intracellular cAMP levels were quantified using the Cyclic AMP XP Assay Kit (Life Technologies, cat. no. 4339). $1 \times 10^5$ cells were pretreated for 30 min with 0.5 mM 3-isobutyl-1-methylxanthine (IBMX, Sigma, cat. no. I5879) in FCS-free DMEM and incubated for another 30 min in FCS-free DMEM adjusted to the desired pH before cAMP levels were determined in cell lysates according to manufacturer's protocol.

PKA inhibition: $1.5 \times 10^5$ cells were pretreated with 10 pM H-89 (Sigma, cat. no. B1472) for 1 h, incubated for 2 h in cell culture medium adjusted to pH 6.9 and maintained for 24 h in cell culture medium adjusted to pH 7.6 before SEAP levels were assessed in the cell culture supernatant

SEAP: Production of human placental secreted alkaline phosphatase was quantified in cell culture supernatants (Schlatter S. et al., Gene 282, 19-31 (2002)) and mouse serum (Weber et. al.,Nat. Biotech., 22, 1440-1444 (2004)) as described before.

Rituximab: The anti-CD20 IgG was quantified using a sandwich ELISA. Wells of a 96-well plate were coated for 16 h at 4°C with AffiniPure Goat Anti-Human IgG,Fc (Jackson Immunoresearch, West Grove, USA; lot. no. 100376). After three washing steps using 250 µL PBS supplemented with 0.15% Tween20, a human IgG standard (Life Technologies, cat. no. 027102; lot no. 1069920A) or samples were added to the wells and incubated for 1.5 h at 22°C, followed by another three washing steps. Then, an alkaline phosphatase-coupled Anti-Human Kappa Light Chain antibody (Sigma; cat. no. A3813, lot no. 071 M6231) was incubated for 1 h followed by a three washing steps. Thereafter, para-nitrophenylphosphate (pNPP; Sigma, cat. no. N2640) was added and absorption was measured at 405 nm (reference 492 nm) using an Infinite® M200 PRO plate reader (Tecan Group Ltd, Männedorf, Switzerland).

Microscopy: Fluorescence and time-lapse microscopy was performed using an inverted fluorescence microscope (DMI 6000B; Leica Microsystems, Heerbrugg, Switzerland) equipped with an incubation chamber (temperature and $CO_2$ control), a DFC350FX R2 digital camera (Leica), a 10x objective (Obj. HC PL FL 10x/0.30 PH1 -/D 11.0; Leica), a 495/535 nm (EYFP) excitation/emission filter set and LAS AF imaging software installed (Leica; FW4000-TZ).

Cell viability: Viability was scored by FACS analysis (Becton Dickinson LSRII Fortessa flow cytometer) of cells that had been double stained with annexin V-FITC (Life Technologies Corporation, cat. no. V1324) and Live/Dead fixable far red dead cell stain kit (Life Technologies Corporation, cat. no. L10120).

Glucose and ketone bodies: To test glucose tolerance of mice, D-glucose was dissolved in sterile $ddH_2O$ (0.25 g/mL) and intraperitoneally injected (1.5 g/kg) into mice that had been fasted overnight. Glucose and ketone bodies were profiled in the blood (glucose and 3-hydroxybutyrate) with a FreeStyle Precision glucometer (Abott Laboratories, Illinois, USA; cat. no. 70085-70) and the urine (glucose and acetoacetic acid) using a Combur® test (Roche Diagnostics GmbH, Basel, Switzerland; cat. no. 0035116, lot. no. 210019-02). Bicarbonate: Serum bicarbonate levels were quantified using a Bicarbonate FL assay (Chema Diagnostica, Monsano, Italy, cat. no. BR F060 CH, lot. no. FY-087).

Insulin: Serum insulin levels were quantified using a Rat/Mouse Insulin ELISA kit (Millipore AG, Zug, Switzerland; cat. no. EZRMI-13K, detection range: 0.1-10 µg/L).

Statistics: Results are shown as mean ± SEM. Statistical significance of data sets was evaluated by a two-tailed, unpaired Student's *t* test using Prism software (GraphPad v5.0a). Differences of $p < 0.05$ were considered statistically significant.

Bioreactor operation

*Freestyle™* 293-F suspension cells were cultivated in a Labfors 5 Lux stirred-tank bioreactor (Infors AG, Bottmingen, Basel, Switzerland) containing 0.6-2.4 L of *FreeStyle™* 293 Expression Medium supplemented with 1% penicillin/streptomycin solution, 1% Pluronic™ F-68 (Life Technologies, cat. no. 24040-032) and 50 ppm Antifoam C Emulsion (Sigma, cat. no. A8011). The pH of cell culture medium was monitored by a pH sensor (Mettler-Toledo, Greifensee, Switzerland) and adjusted by influx of carbon dioxide and 1 M NaOH. All culture parameters were set, monitored and controlled by the Labfors and IRIS software (Infors AG; Labfors v1.2.5.518, IRIS v5.3): temperature, 37°C; pH, user-defined using $CO_2$ and 1 M NaOH (6.8-7.6); Dissolved oxygen (DO), 40% of air saturation; aeration rate, coupled *via* a cascade to DO (50-150 mL/min); stirrer speed, coupled *via* a cascade to Flow (20-70 rpm). Samples were taken with a Super Safe Sampler (Infors AG, Bottmingen, Basel, Switzerland).

Implant production

Cell implants were produced by encapsulating pDA62/pCK53- or pDA62/pDA145-transgenic HEK-293T into coherent alginate-(poly-L-lysine)-alginate capsules (400 μm, 200 cells/capsule) using an Inotech Encapsulator Research IE-50R (EncapBioSystems, Greifensee, Switzerland) set to the following parameters: 0.2 mm single nozzle, stirrer speed control 5 units, 20 mL syringe with a flow rate of 410 units, nozzle vibration frequency of 1,024 Hz, 900V for capsule dispersion. Integrity and quality of capsules maintained at different pH was confirmed by microscopy.

Animal experiments

For simulation of a metabolic acidosis in wild-type mice, female Swiss Mice (Oncins France souche 1, Charles River, Laboratories Lyon, France) received twice daily intraperitoneal injections of acetazolamide (Sigma, cat. no. A6011; 200 mg/kg in 300 μL PBS) during 5 days. To establish the type 1 diabetes animal model the mice were fasted for 36 h and injected with a single dose of alloxan monohydrate (Sigma, cat. no. A7413, 200 mg/kg in 300 μL PBS), while the control treatment group received PBS injections (Federiuk, I.F. et al., 2004, Comparative Med 54, 252-257). Mice that developed type 1 diabetes and diabetic ketoacidosis after 48 h showed high glucose levels in the blood (>20 mmol/L) and urine (>55 mmol/L) as well as elevated concentrations of ketone bodies in the blood (3-hydroxybutyrate >2 mmol/L) and the urine (acetoacetic acid >15 mmol/L). Mice were intraperitoneally implanted with 2-5x10^6 microencapsulated transgenic cells (1x10^4 capsules in 0.7 ml serum-free DMEM). After 24 h blood samples were collected and the serum isolated using microtainer SST tubes (Becton Dickinson, New Jersey, USA) according to the manufacturer's protocol. All experiments involving mice were performed according to the directives of the European Community Council (86/609/EEC), approved by the French Republic (N°69266309) and carried out by Ghislaine Charpin-El Hamri at the Institut Universitaire de Technology, IUTA, F-69622 Villeurbanne Cedex, France.

Model for $CO_2$-control-mediated transgene induction

The pH value of bicarbonate-buffered cell culture media correlates with the carbon dioxide concentration in the cell culture incubator atmosphere. This correlation is represented by the Henderson-Hasselbalch equation:

$$pH = 6.1 + \log\left(52\frac{[NaHCO_3]}{[CO_2]} - 1\right) = f_{pH}\left([NaHCO_3], [CO_2]\right) \qquad (1)$$

where $[NaHCO_3]$ is given in [g/L], $[CO_2]$ in [%] and 6.1 denotes the pKa value of carbonic acid. The cell culture pH resulting from various combinations of bicarbonate (0.2-4 g/L) and $CO_2$ (1-14%) concentrations is shown in Fig. 5A.

To simulate $CO_2$-control-mediated transgene induction ($CO_2$TI) as a function of bicarbonate and carbon dioxide a standard sigmoidal dose-response equation was applied with proton molecules as the agonist:

$$CO_2TI\left([NaHCO_3], [CO_2]\right) = Bottom + \left(\frac{Top - Bottom}{1 + 10^{(\log EC50 - pH) \times Hillcoefficient}}\right) \qquad (2)$$

where Bottom and Top equals the lowest (0%) and highest (100%) level of $CO_2$-control-mediated transgene induction, $\log EC_{50}$ refers to the logarithm of agonist concentration leading to 50% of $CO_2$-control-mediated transgene induction, the Hill coefficient represents the slope of the dose-response curve and pH represents the proton concentration that introduces a functional correlation of $[NaHCO_3]$ and $[CO_2]$ according to equation (1).

The parameters $\log EC_{50}$ and HillSlope were experimentally determined by cultivating pDA62-/pCK53-cotransfected HEK-293T in medium adjusted to different pH and profiling resulting SEAP production levels. This resulted in a proton dose-responsive SEAP expression profile that was fitted using equation (2) to reveal $logEC_{50}$ = 7.15 and HillSlope = -5.584 (Fig. 2B).

**Claims**

1. A mammalian cell comprising

   (a) a vector expressing a pH sensing receptor comprising a promoter and a polynucleotide coding for a G protein-coupled receptor;
   (b) a vector comprising a synthetic promoter specifically activated by said pH sensing receptor and response elements fused to a polynucleotide coding for an endogenous or exogenous protein.

2. The mammalian cell according to claim 1 wherein the G protein-coupled receptor is TDAG8, OGR1 or GPR4 or a related receptor.

3. The mammalian cell according to claim 1 wherein the G protein-coupled receptor is TDAG8, OGR1 or GPR4.

4. The mammalian cell according to claim 1 wherein the G protein-coupled receptor is TDAG8.

5. The mammalian cell according to claim 1 wherein the encoded endogenous or exogenous protein is a drug protein or a therapeutic antibody.

6. The mammalian cell according to claim 1 wherein the encoded endogenous or exogenous protein is a carbonic anhydrase, insulin, leptin, glucagon-like peptide 1 (glp-1), glucagon, a detoxifying enzyme, or a therapeutic antibody.

7. The mammalian cell according to claim 1 wherein the encoded endogenous or exogenous protein is insulin or the therapeutic antibody rituximab.

8. A vector comprising a synthetic promoter specifically activated by a pH sensing G protein-coupled receptor and a response element fused to a polynucleotide coding for an endogenous or exogenous protein.

9. The vector according to claim 8 wherein the promoter is the constitutive simian virus 40 promoter ($P_{SV40}$), the minimal human cytomegalovirus immediate early promoter ($P_{hCMVmin}$), the constitutive human cytomegalovirus promoter ($P_{hCMV}$), the human elongation factor $1\alpha$ promoter ($P_{hEF1\alpha}$), the phosphoglycerate kinase promoter ($P_{PGK}$), the human ubiquitin promoter ($P_{hUBC}$), or the beta-actin promoter.

10. The vector according to claim 8 wherein the response element is the cAMP-response element (CRE), the nuclear factor of activated T cells (NFAT) response element, the serum response element (SRE) or the 12-O-tetradecanoyl-phorbol-13-acetate response element (TRE).

11. A method for the $CO_2$-inducible production of biopharmaceuticals wherein a mammalian cell according to any of claims 1 to 7 expressing the biopharmaceutical is cultured in a bioreactor with pH control by introducing $CO_2$.

12. A mammalian cell according to claims 1 to 7 in a nano- or microcontainer.

13. A mammal excluding man comprising a mammalian cell according to claims 1 to 7 or 12.

14. A method of treating acidosis-related disorders and type 1 diabetes comprising administering to a patient in need thereof a therapeutically effective amount of a mammalian cell in a nano- or microcontainer according to claim 12.

**Fig. 1**

**Fig. 2 A**

**Fig. 2 B, C**

**Fig. 2 D, E**

**Fig. 2 F**

**Fig. 3**

## Fig. 4

pDA62 (P$_{SV40}$-TDAG8-pA)

pCK91 (P$_{CRE}$-eYFP-pA)

**Fig. 5 A, B**

**A**

**B**

**Fig. 5 C, D**

**Fig. 5 E, F**

**Fig. 6**

**Fig. 7 A**

**A**

**Fig. 7 B, C**

**B**

**C**

Fig. 8

**Fig. 9**

**A**

pH 7.6

pH 6.9

**B**

**Fig. 10**

**Fig. 11 A, B**

**A**

**B**

**Fig. 11 C, D**

**Fig. 11 E**

**EUROPEAN SEARCH REPORT**

Application Number

EP 13 00 0700

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SIN WUN CHEY ET AL: "G protein-coupled receptors GPR4 and TDAG8 are oncogenic and overexpressed in human cancers", ONCOGENE, vol. 23, no. 37, 19 August 2004 (2004-08-19), pages 6299-6303, XP002699841, ISSN: 0950-9232 * figure 2 * | 1-14 | INV. C12N15/63 |
| X | DE VALLIERE CHERYL ET AL: "Functional Consequences of G Protein-Coupled Receptor 68 (GPR68/OGR1) Overexpression in Intestinal Epithelial Cells", GASTROENTEROLOGY, vol. 142, no. 5, Suppl. 1, May 2012 (2012-05), page S809, XP009170596, & DIGESTIVE DISEASE WEEK (DDW); SAN DIEGO, CA, USA; MAY 19 -22, 2012 * the whole document * | 1-14 | |
| X | TOBO ET AL: "Previously postulated ''ligand-independent'' signaling of GPR4 is mediated through proton-sensing mechanisms", CELLULAR SIGNALLING, ELSEVIER SCIENCE LTD, GB, vol. 19, no. 8, 21 June 2007 (2007-06-21), pages 1745-1753, XP022124776, ISSN: 0898-6568, DOI: 10.1016/J.CELLSIG.2007.03.009 * paragraphs [02.8], [03.3] * | 1-14 | TECHNICAL FIELDS SEARCHED (IPC) C12N |

-/--

The present search report has been drawn up for all claims

| Place of search | Date of completion of the search | Examiner |
|---|---|---|
| Munich | 8 July 2013 | Mabit, Hélène |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

Europäisches
Patentamt

European
Patent Office

Office européen
des brevets

**EUROPEAN SEARCH REPORT**

Application Number

EP 13 00 0700

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | YE HAIFENG ET AL: "A Synthetic Optogenetic Transcription Device Enhances Blood-Glucose Homeostasis in Mice", SCIENCE (WASHINGTON D C), vol. 332, no. 6037, June 2011 (2011-06), pages 1565-1568, XP002699843, ISSN: 0036-8075 ----- | 1-14 | |
| A | RADU CAIUS G ET AL: "Differential proton sensitivity of related G protein-coupled receptors T cell death-associated gene 8 and G2A expressed in immune cells", PROCEEDINGS OF THE NATIONAL ACADEMY OF SCIENCES OF THE UNITED STATES OF AMERICA, vol. 102, no. 5, 1 February 2005 (2005-02-01), pages 1632-1637, XP002699844, ISSN: 0027-8424 ----- | 1-14 | |

TECHNICAL FIELDS
SEARCHED (IPC)

The present search report has been drawn up for all claims

| Place of search | Date of completion of the search | Examiner |
|---|---|---|
| Munich | 8 July 2013 | Mabit, Hélène |

CATEGORY OF CITED DOCUMENTS

X : particularly relevant if taken alone
Y : particularly relevant if combined with another
  document of the same category
A : technological background
O : non-written disclosure
P : intermediate document

T : theory or principle underlying the invention
E : earlier patent document, but published on, or
  after the filing date
D : document cited in the application
L : document cited for other reasons

& : member of the same patent family, corresponding
  document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **WEBER W. ; FUSSENEGGER M.** *Nat Rev Genet,* 2012, vol. 13, 21-35
- **HOVORKA R.** *Nat Rev Endocrinol,* 2011, vol. 7, 385-395
- **KITABCHI A.E. et al.** *Diabetes Care,* 2001, vol. 24, 131-153
- **CASEY J.R. et al.** *Nat Rev Mol Cell Biol,* 2010, vol. 11, 50-61
- **AGGARWAL S.** *Nature Biotechnology,* 2011, vol. 29, 1083-1089
- **ISHII S. et al.** *J Biol Chem,* 2005, vol. 280, 9083-9087
- **DAYHOF, M.D.** *Nat. Biomed. Res. Found.,* 1978, vol. 5 (3
- **HAFNER P. et al.** *Am J Physiol-Cell Ph,* 2008, vol. 295, C1658-C1667
- **BRENT J.** *N Engl J Med,* 2009, vol. 360, 2216-2223
- **BARKER J.M. et al.** *Diabetes Care,* 2004, vol. 27, 1399-1404
- **CHESON B.D. ; LEONARD J.P.** *N Engl J Med,* 2008, vol. 359, 613-626
- **HAY C.W. ; DOCHERTY K.** *J Mol Endocrinol,* 2003, vol. 31, 597-607
- **YE H. et al.** *Science,* 2011, vol. 332, 1565-1568
- **KEMMER C. et al.** *Nat Biotechnol,* 2010, vol. 28, 355-360
- **KEMMER C. et al.** *J Control Release,* 2011, vol. 150, 23-29
- **SIMONSEN, J.L. et al.** *Nat Biotechnol,* 2002, vol. 20, 592-596
- **SCHLATTER S. et al.** *Gene,* 2002, vol. 282, 19-31
- **WEBER.** *Nat. Biotech.,* 2004, vol. 22, 1440-1444
- **FEDERIUK, I.F. et al.** *Comparative Med,* 2004, vol. 54, 252-257